(19) **Europäisches Patentamt / European Patent Office / Office européen des brevets**

(11) **EP 2 478 897 B1**

(12) **EUROPEAN PATENT SPECIFICATION**

(45) Date of publication and mention
of the grant of the patent:
**07.03.2018 Bulletin 2018/10**

(21) Application number: **10815661.3**

(22) Date of filing: **14.09.2010**

(51) Int Cl.:
*A61K 9/70* (2006.01)     *A61K 47/36* (2006.01)

(86) International application number:
**PCT/KR2010/006250**

(87) International publication number:
**WO 2011/031116 (17.03.2011 Gazette 2011/11)**

(54) **PAD FOR HERBAL MEDICINE IN WHICH RELEASE OF MEDICINAL INGREDIENT CAN BE CONTROLLED, AND MANUFACTURING METHOD THEREOF**

AUFLAGE FÜR KRÄUTERMEDIZIN MIT STEUERBARER FREISETZUNG DES WIRKSTOFFES UND HERSTELLUNGSVERFAHREN DAFÜR

COMPRESSE DE MÉDICAMENT À BASE DE PLANTE DANS LAQUELLE LA LIBÉRATION D'INGRÉDIENT MÉDICINAL PEUT ÊTRE RÉGULÉE, ET SON PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **14.09.2009 KR 20090086728**

(43) Date of publication of application:
**25.07.2012 Bulletin 2012/30**

(73) Proprietors:
- **Kim, Hi Gu**
  **Masanhoewon-gu, Changwon-si**
  **Gyeongsangnam-do (KR)**
- **BM Biotechnology Co., Ltd.**
  **Uichang-gu,**
  **Changwon-si, Gyeongsangnam-do (KR)**

(72) Inventor: **Kim, Hi Gu**
**543-1 Sinyong-dong,**
**Buk-gu, Gwangju (KR)**

(74) Representative: **Dr. Gassner & Partner mbB**
**Marie-Curie-Str. 1**
**91052 Erlangen (DE)**

(56) References cited:
WO-A1-2004/110428     WO-A1-2005/013943
CN-A- 100 998 741      CN-A- 101 199 745
CN-A- 101 278 979      KR-A- 19980 066 346
KR-A- 20020 003 462    KR-A- 20020 021 314
KR-A- 20050 116 503

**Description**

[Technical Field]

The present disclosure relates to a pad for herbal medicine and a method for manufacturing the same. More particularly, it relates to a pad for herbal medicine allowing for persistent supply of medical ingredients through skin or respiratory organs and a method for manufacturing the same.

[Background Art]

In general, drug delivery through the skin is achieved by electrophoresis or electroosmosis. The drug administered transdermally in this manner reaches the bloodstream, being aided by ions. In particular, in case of electroosmosis, aqueous solution is attracted to the negative (-) electrode and current flows via membrane with small pores.
Drug administration using devices related to these techniques is quite limited. US Patent Nos. 2,493,155, 4,141,359, 4,250,878, 3,163.166, 4,166,457, 4,273,135, 3,289,671, 4,239,052, 4,367,745, 3,677,268 and 4,243,052 disclose methods for administering drugs through skin.
Particularly, it is to be noted that, according to US Patent Nos. 3,289,671 and 4,141,359, the rate of drug administration is a function of current and the control of current is critical in controlling the amount of drug administration. US Patent No. 588,479 discloses an electric herb pad providing electrical effect as well as herbal medicinal effect at the same time for human body.
Iontophoresis is a transdermal drug administration technique capable of complementing or replacing existing oral administration or injection. Differently from the passive drug transport using a pad attached to the skin, it enables active transport of soluble drug through the skin using electric induction. In an iontophoretic apparatus, a drug pad containing drug is attached to the skin. Then magnetic or electrical stimulation is applied to the drug pad such that the drug pad is maintained in a positively or negatively charged state and the drug is penetrated into the skin owing to electrical or magnetic repulsion. The iontophoretic technique is used in cooling or heating pads for drug delivery.
Korean Patent No. 0775675 discloses a vibration pad to stimulate the body for physiotherapy and an apparatus for controlling the same.
The pad is configured such that the drug ingredient is easily absorbed into the skin by applying electric field or magnetic field to a medicinal ingredient layer formed in the pad.
However, the patent focuses only on the fast penetration of drug without considering the persistence of the drug administration through the skin and, thus, it is difficult to persistently administer the drug through the skin. Moreover, it is impossible to vary the rate of penetration depending on the particular drug included in the medicinal ingredient layer. CN 101278979 A discloses a herbal medicine patch and a method for preparing it wherein the medicinal ingredients are ground into powder.
When preparing herbal medicine from plants by mixing different ingredients obtained from, for example, the whole plant, leaves, root and rhizome, fruit and seed, flower, bark, stem, or the like, it may be necessary to control the time or amount of penetration into the skin according to pharmacological effect so as to achieve persistent drug delivery, which is difficult to be achieved with existing pads or patches.

**[Disclosure]**

[Technical Problem]

The present disclosure is directed to solving the above-described problems and providing a pad for herbal medicine in which the release and penetration of medicinal ingredients to the skin can be controlled differently for different medical ingredients so as to maximize the therapeutic effect and a method for manufacturing the same.
The present disclosure is also directed to providing a pad for herbal medicine in which the release of medicinal ingredients can be controlled by applying electrical stimulation or magnetic force to the pad to improve the efficiency of penetration into the skin and a method for manufacturing the same.
The present disclosure is also directed to providing a pad for herbal medicine in which the release of medicinal ingredients can be controlled allowing for maintenance of physical properties of two or more herbal medicinal ingredients prescribed according to pharmacological effects, long-term storage of the ground herbal medicinal ingredients at room temperature, control of release amount and release duration of the medicinal ingredients and maximized delivery effect of the medical ingredients via skin or respiratory organs, and a method for manufacturing the same.

[Technical Solution]

In one general aspect, the present disclosure provides a method for manufacturing a pad for herbal medicine in which the release of medicinal ingredients can be controlled, which comprises:

separating two or more medicinal ingredients prescribed or prepared according to pharmacological effects on the basis of the release duration of the medicinal ingredients;
grinding the separated medicinal ingredients to fine particles with different sizes on the basis of the release duration of the medicinal ingredients;
preparing herbal medicine by mixing the ground medicinal ingredients with a binding agent; and
adhering the prepared herbal medicine to a base sheet.

In an exemplary embodiment of the present disclosure, the binding agent may be maltodextrin or natural polymer.
In the step of preparing the herbal medicine, a step of providing a medicinal ingredient permeation activating unit of providing a magnet or a low frequency generator for applying magnetic field or low frequency to the medicinal ingredients of the pad may be further included.
In another general aspect, the present disclosure provides a pad for herbal medicine comprising:

a base sheet; and
a medicinal ingredient layer adhered to the base sheet, in which two or more medicinal ingredients prescribed or prepared according to pharmacological effects on the basis of the release duration of the medicinal ingredients which are ground to fine particles of different sizes according to the setting of release duration are mixed with a binding agent comprising maltodextrin or natural polymer.

The pad may further comprise a medicinal ingredient permeation activating unit provided on base sheet or between the medicinal ingredient layer and the base sheet, and the medicinal ingredient permeation activating unit may comprise a magnet or a low frequency generator.

[Advantageous Effects]

The pad for herbal medicine and the method for manufacturing the same according to the present disclosure allow for persistent permeation of medical ingredients through the skin with different release rate for each medicinal ingredient, and thus, the efficacy of the medicinal ingredient layer and the effect of treating disease can be maximized.
Since the pad according to the present disclosure can be manufactured as a mask, necklace, bracelet, or the like, for penetration of medicinal ingredients into the skin, treatment may be achieved more conveniently and the commercial value may be enhanced.
In addition, the pad for herbal medicine of the present disclosure may be attached to a mask, necklace, bracelet, compression pad, etc. for treatment purposes so as to deliver prescribed herbal medicinal ingredients. Since the pad for herbal medicine can deliver medicinal ingredient through the skin, it may be developed into various products.

[Description of Drawings]

Fig. 1 is a schematic perspective view of a pad for herbal medicine according to the present disclosure.
Fig. 2 is a block diagram illustrating a method for manufacturing a pad for herbal medicine according to the present disclosure.

[Best Mode]

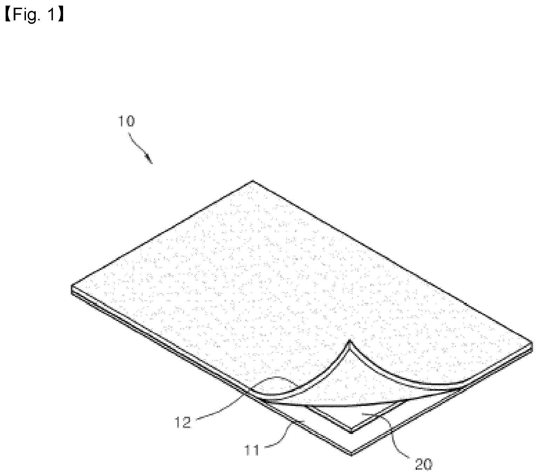

A pad for herbal medicine according to the present disclosure allows medicinal ingredients to penetrate into the skin with controlled penetration time, i.e. release duration, for treatment of diseases. An exemplary embodiment is shown in Fig. 1.
Referring to the figure, a pad 10 according to the present disclosure comprises a base sheet 11 comprising non-woven cloth, a medicinal ingredient layer 12 having a predetermined thickness and spread to the base sheet 11, and a medicinal ingredient permeation activating unit 20 provided on base sheet 11 or between the medicinal ingredient layer 12 and the base sheet 11, the medicinal ingredient permeation activating unit 20 comprising a magnet or a low frequency generator for applying magnetic field or low frequency.

The base sheet 11 may comprise non-woven cloth, but is not limited thereto. For example, the base sheet may comprise synthetic resin, natural fiber, paper, natural nonwoven, or the like. In particular, the base sheet 11 may be further provided with a magnet powder layer as a medicinal ingredient permeation activating unit.

The medicinal ingredient layer 12 comprises two or more medicinal ingredients prescribed or prepared according to pharmacological effects on the basis of the release duration of the medicinal ingredients which are ground to fine particles of different sizes according to the setting of penetration time, i.e. release duration, and mixed (or encapsulated) with a binding agent. When a relatively long release duration is desired, the medical ingredient may be ground to relatively large particle size (10-400 $\mu$m), and, when a relatively short release duration is desired, the medical ingredient may be ground to have a particle size of 10-15 $\mu$m. For example, when the medical ingredient is desired to be released for a duration of from 1 day to 1 year or longer, the medical ingredient may be ground to have a particle size of from 10 $\mu$m up to 400 $\mu$m. And, when two or more medical ingredients are desired to be released persistently until the release of all the medical ingredients is completed, the medical ingredients may be ground to fine particles of different sizes from 10 $\mu$m up to 400 $\mu$m based on their relative weight, so that the medical ingredient of small amount has a larger size and that with a large amount has a smaller size.

Specifically, the binding agent which encapsulates the ground medical ingredient may comprise maltodextrin or natural polymer which is capable of preventing decomposition of the medical ingredient for a long period of time and holding moisture. The binding agent may be used in an amount of not greater than 40% (40/100) of the total weight of the ground herbal medicine. When the binding agent is mixed with the ground herbal medicine, the mixing speed may be 100 rpm or lower. And, when honey, which is a natural polymer, is used as the binding agent, the amount may be not greater than 80-90 parts by weight based on 100 parts by weight of the ground herbal medicine, considering the water-holding ability of honey.

The medicinal ingredient permeation activating unit 20 is capable of activating the permeation of the medicinal ingredient into the skin by applying electric field or magnetic field to the medicinal ingredient layer. A magnetic substance capable of applying a magnetic force of 2500-7000 gauss may be provided between the medicinal ingredient layer 12 and the base sheet 11. The magnetic substance may comprise a rubber magnet, and may be provided as the magnet powder layer of the base sheet as described above or may be embedded or mixed as magnet powder in the base sheet.

In another exemplary embodiment, the medicinal ingredient permeation activating unit may comprise a low frequency generator (not shown) provided on one side of the base sheet and a low frequency transport layer connected to the low frequency generator and embedded in the medicinal ingredient layer. Without being limited to the above-described embodiments, the medicinal ingredient permeation activating unit may be of any configuration capable of activating the penetration of the medicinal ingredient into the skin. For example, a light-emitting diode radiating light of specific wavelength region to the target site may be used.

In addition, an auxiliary sheet having a number of holes may be attached on the medicinal ingredient layer. Also, a protection sheet for protecting the medicinal ingredient layer until use may be further provided.

The pad for herbal medicine may be used after being attached to a mask, necklace, bracelet, compression pad, or the like. When it is attached to a bracelet, compression pad or necklace, the pad for herbal medicine may be disposed such that it contacts with the skin.

The pad for herbal medicine comprising two or more medicinal ingredients prescribed or prepared according to pharmacological effects may be modified to suit the site of application and be attached thereto so as to achieve controlled release duration of the medical ingredient according to purposes such as treatment of neuralgia, arthritis, shoulder stiffness, etc.

The particle size of the two or more medicinal ingredients may be determined by the following equation. The equation is based on the physical principle that the smaller the particle size the shorter is the release duration, and the larger the particle size the longer is the release duration.

$$\log \frac{S}{S_o} = \frac{2\gamma V}{2.303 RTr}$$

$S$: release amount of relatively small-sized particles, $S_o$: release amount of relatively large-sized particles, $\gamma$: surface tension of particles, $V$: molecular volume (cm$^3$), $r$: final diameter (cm) of particles, $R$: gas constant (8.314x10$^7$ erg/deg mol), $T$: absolute temperature.

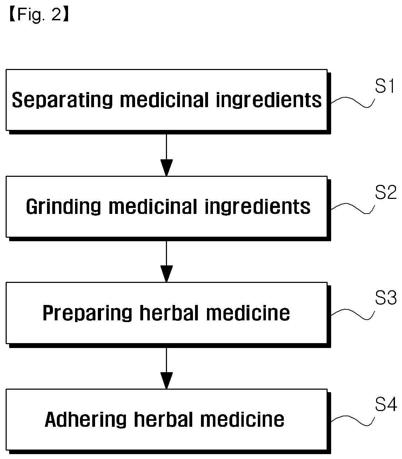

A method for manufacturing the pad for herbal medicine comprises, as shown in Fig. 2, separating two or more medicinal ingredients prescribed or prepared according to pharmacological effects on the basis of the release duration of the medicinal ingredients into the skin (S1) and grinding the separated medicinal ingredients to fine particles with different sizes on the basis of the release duration of the medicinal ingredients (S2). The particle size of the finely ground medicinal ingredients may be determined according to the weight proportion of the medicinal ingredients. Specifically, a medicinal ingredient with a smaller weight proportion may be ground to have a relatively larger particle size.

The method for manufacturing the pad for herbal medicine further comprises preparing herbal medicine by mixing the ground medicinal ingredients with a binding agent (S3) and adhering the prepared herbal medicine to a base sheet (S4). The step of preparing the herbal medicine may comprise mixing the ground medicinal ingredients with a binding agent comprising honey, maltodextrin or natural polymer such that the medicinal ingredients are encapsulated by the binding agent. In the adhering step, the herbal medicine is coated on the base sheet. It may further comprise providing a medicinal ingredient permeation activating unit comprising a magnet or a low frequency generator for applying magnetic field or low frequency to the medicinal ingredients of the pad.

The present disclosure is described in more detail by the following test examples. However, the scope of present disclosure is not limited by the test examples.

Test Example 1

Release duration of medicinal ingredients prescribed for skin disease and inflammation having different particle sizes was tested. Herbal medicine was prepared from red bean (3 g), apricot kernel (4 g), ephedra, forsythia, ginger, jujube and mulberry root (3 g each) and licorice (1 g).

The apricot kernel was ground to a particle size of 10 $\mu$m (minimum particle size required for maintaining the physical properties of the herbal medicine), the red bean, ephedra, forsythia, jujube and mulberry root were ground to a particle size of 15 $\mu$m, and the licorice was ground to a particle size of 40 $\mu$m.

The ground medicinal ingredients were mixed with 80 parts by weight of honey based on the total weight of the ground medicinal ingredients and applied on a base sheet to form a 5mm thick medicinal ingredient layer.

Thus prepared pad for herbal medicine was cut to a sample of 50 mm x 50 mm size and adhered to the disease site. Then, the rate and time of release of the medicinal ingredients from the medicinal ingredient layer were measured. The drug release amount was measured by atomic analysis of the medical ingredients after a given period of time.

The release amount from the medicinal ingredient layer was measured 24 hours after attaching the pad on the knee. At 24hours, the release amount was 45% and 40% of initial release for red bean and apricot kernel, respectively. The release amount of ephedra, forsythia, jujube and mulberry root was 45% of initial release. And, the release amount of licorice with a relatively larger particle size was 40% of initial release. As seen from Table 1, the time and amount of release of the medicinal ingredients are determined by the particle size, without regard to the content of the medicinal ingredients.

Table 1

|  | Weight (g) of medicinal ingredient | Particle size ($\mu$m) | Release ratio (%) | Note |
|---|---|---|---|---|
| Licorice | 1 | 40 | 40 | |
| Red bean | 3 | 15 | 45 | |
| Ephedra | 3 | 15 | 45 | |
| Forsythia | 3 | 15 | 45 | |
| Jujube | 3 | 15 | 45 | |
| Mulberry root | 3 | 15 | 45 | |
| Ginger | 3 | 15 | 45 | |
| Apricot kernel | 4 | 10 | 40 | |

Test Example 2

Herbal medicine was prepared as in Test Example 1. The red bean, apricot kernel, ephedra, forsythia, jujube and mulberry root were ground to a particle size of 20 $\mu$m, and the licorice was ground to a particle size of 25 $\mu$m.

The ground medicinal ingredients were mixed with 90 parts by weight of honey based on the total weight of the ground medicinal ingredients and applied on a base sheet to form a 5-mm thick medicinal ingredient layer.

The release from the medicinal ingredient layer was determined 7 days after attaching the pad on the knee. The release amount of red bean was 10% of initial release, and the release amount of ephedra, forsythia, jujube and mulberry root was 10.3% of initial release. The release amount of licorice with a relatively larger particle size was 20% of initial release.

Test Example 3

Herbal medicine was prepared as in Test Example 1. The red bean was ground to a particle size of 370 μm, the apricot kernel, ephedra, forsythia, jujube and mulberry root were ground to a particle size of 375 μm, and the licorice was ground to a particle size of 300 μm.
The release from the medicinal ingredient layer was determined 7 days after attaching the pad on the knee. The release amount of red bean was 11% of initial release, and the release amount of ephedra, forsythia, jujube and mulberry root was 11.2% of initial release. The release amount of licorice with a relatively larger particle size was 5% of initial release.

Test Example 4

Experiment was performed under the same condition as in Test Example 1 after attaching a magnet of 2,600 gauss to the base sheet.
The release from the medicinal ingredient layer was determined 24 hours after attaching the pad on the knee. The release amount of red bean was 3% of initial release, and the release amount of ephedra, forsythia, jujube and mulberry root was 3.5% of initial release. The release amount of licorice with a relatively larger particle size was 3% of initial release.

**Claims**

1. A method for manufacturing a pad for herbal medicine allowing for persistent supply of herbal medical ingredients through skin or respiratory organs, wherein the ingredients are present in different amounts and released persistently until release of all the medical ingredients is completed, in which pad the release of the medicinal ingredients can be controlled, which comprises: separating two or more medicinal ingredients prescribed or prepared according to pharmacological effects on the basis of the release duration of the medicinal ingredients to be delivered via skin or respiratory organs; grinding the separated medicinal ingredients to fine particles with different sizes from 10 μm up to 400 μm on the basis of the release duration of the medicinal ingredients and their relative weights, so that a medical ingredient of smaller amount has a larger size and a medical ingredient with a larger amount has a smaller size; preparing herbal medicine by mixing the ground medicinal ingredients with a binding agent comprising maltodextrin or natural polymer; and adhering the herbal medicine in a medicinal ingredient layer to a base sheet.

2. The method for manufacturing a pad for herbal medicine in which the release of medicinal ingredients can be controlled of Claim 1, wherein said preparing the herbal medicine further comprises providing a medicinal ingredient permeation activating unit comprising a magnet or a low frequency generator for applying magnetic field or low frequency to the medicinal ingredients of the pad.

3. The method of claim 1 or 2, wherein said base sheet is provided with a magnet powder layer as a/said medicinal ingredient permeation activating unit.

4. The method of claim 3, wherein said magnet powder layer comprises a rubber magnet.

5. The method of any of the preceding claims, wherein a/said medicinal ingredient permeation activating unit is provided between the medicinal ingredient layer and the base sheet.

6. The method of any of the preceding claims, wherein the binding agent is used in an amount of not greater than 40% (40/100) of the total weight of the ground medicinal ingredients.

7. The method of any of the preceding claims, wherein said binding agent comprises honey.

8. A pad for herbal medicine allowing for persistent supply of herbal medical ingredients through skin or respiratory organs, wherein the ingredients are present in different amounts and released persistently until release of all the medical ingredients is completed, in which pad the release of the medicinal ingredients can be controlled, which comprises: a base sheet; and a medicinal ingredient layer adhered to the base sheet, in which two or more medicinal ingredients prescribed or prepared according to pharmacological effects on the basis of the release duration of the medicinal ingredients to be delivered via skin or respiratory organs are mixed with a binding agent comprising maltodextrin or natural polymer, which medicinal ingredients are ground to fine particles of different sizes from 10

μm up to 400 μm according to the setting of release duration of the medicinal ingredients and their relative weights, so that a medical ingredient of smaller amount has a larger size and a medical ingredient with a larger amount has a smaller size.

9. The pad for herbal medicine in which the release of medicinal ingredients can be controlled of Claim 8, which further comprises a medicinal ingredient permeation activating unit provided on base sheet or between the medicinal ingredient layer and the base sheet, the medicinal ingredient permeation activating unit comprising a magnet or a low frequency generator.

10. The pad of claim 8 or 9, wherein said base sheet is provided with a magnet powder layer as a/said medicinal ingredient permeation activating unit.

11. The pad of claim 10, wherein said magnet powder layer comprises a rubber magnet.

12. The pad of any of claims 8 to 11, wherein a/said medicinal ingredient permeation activating unit is provided between the medicinal ingredient layer and the base sheet.

13. The pad of any of claims 8 to 12, wherein the amount of binding agent is not greater than 40% of the total weight of the ground medicinal ingredients.

14. The pad of any of claims 8 to 13, wherein said binding agent comprises honey.

**Patentansprüche**

1. Verfahren zur Herstellung einer Kompresse für Kräutermedizin, welche eine ständige Versorgung mit Bestandteilen der Kräutermedizin durch Haut oder Atmungsorgane ermöglicht, wobei die Bestandteile in unterschiedlichen Mengen vorliegen und ständig freigesetzt werden, bis eine Freisetzung aller der medizinischen Bestandteile beendet ist, wobei in der Kompresse die Freisetzung der medizinischen Bestandteile gesteuert werden kann, umfassend: Trennen von zwei oder mehr verordneten oder nach pharmakologischen Wirkungen hergestellten medizinischen Bestandteilen auf der Basis der Freisetzungsdauer der über die Haut oder Atmungsorgane zuzuführenden medizinischen Bestandteile; Mahlen der abgesonderten medizinischen Bestandteile zu feinen Partikeln mit unterschiedlichen Größen von 10 μm bis zu 400 μm auf der Basis der Freisetzungsdauer der medizinischen Bestandteile und ihrer relativen Gewichte, so dass ein medizinischer Bestandteil kleinerer Menge eine größere Größe aufweist und ein medizinische Bestandteil mit einer größeren Menge eine geringere Größe aufweist; Zubereiten der Kräutermedizin durch Mischen der gemahlenen medizinischen Bestandteile mit einem Maltodextrin oder ein natürliches Polymer umfassenden Bindemittel; und Binden der Kräutermedizin in einer Medizinbestandteilschicht an eine Basislage.

2. Verfahren zur Herstellung einer Kompresse für Kräutermedizin, in welcher die Freisetzung medizinischer Bestandteile gesteuert werden kann, nach Anspruch 1, wobei das Zubereiten der Kräutermedizin weiterhin ein Bereitstellen einer Aktivierungseinheit für ein Eindringen eines medizinischen Bestandteils, umfassend einen Magneten oder einen Niederfrequenz-Generator zum Anwenden eines magnetischen Felds oder einer Niederfrequenz auf die medizinischen Bestandteile der Kompresse, umfasst.

3. Verfahren nach Anspruch 1 oder 2, wobei die Basislage mit einer Magnetpulverschicht als eine/die Aktivierungseinheit für ein Eindringen eines medizinischen Bestandteils ausgestattet ist.

4. Verfahren nach Anspruch 3, wobei die Magnetpulverschicht einen Gummimagneten umfasst.

5. Verfahren nach einem der vorhergehenden Ansprüche, wobei ein/die Aktivierungseinheit für ein Eindringen eines medizinischen Bestandteils zwischen der Medizinbestandteilschicht und der Basislage bereitgestellt wird.

6. Verfahren nach einem der vorhergehenden Ansprüche, wobei das Bindemittel in einer 40% (40/100) des Gesamtgewichts der gemahlenen medizinischen Bestandteile nicht übersteigenden Menge verwendet wird.

7. Verfahren nach einem der vorhergehenden Ansprüche, wobei das Bindemittel Honig umfasst.

8. Kompresse für Kräutermedizin, welche eine ständige Versorgung mit Bestandteilen der Kräutermedizin durch Haut oder Atmungsorgane ermöglicht, wobei die Bestandteile in unterschiedlichen Mengen vorliegen und ständig freigesetzt werden, bis eine Freisetzung aller der medizinischen Bestandteile beendet ist, wobei in der Kompresse die Freisetzung der medizinischen Bestandteile gesteuert werden kann, umfassend: eine Basislage; und eine an eine Basislage gebundene Medizinbestandteilschicht, in welcher zwei oder mehr verordnete oder nach pharmakologischen Wirkungen hergestellte medizinische Bestandteile auf der Basis der Freisetzungsdauer der über Haut oder Atmungsorgane zuzuführenden medizinischen Bestandteile mit einem Maltodextrin oder ein natürliches Polymer umfassenden Bindemittel gemischt sind, wobei die medizinischen Bestandteile zu feinen Partikeln unterschiedlicher Größen von 10 $\mu$m bis zu 400 $\mu$m gemäß dem Festlegen der Freisetzungsdauer der medizinischen Bestandteile und ihrer relativen Gewichte gemahlen sind, so dass ein medizinischer Bestandteil von kleinerer Menge eine größere Größe aufweist und ein medizinischer Bestandteil mit einer größeren Menge eine kleinere Größe aufweist.

9. Kompresse für Kräutermedizin, in welcher die Freisetzung medizinischer Bestandteile gesteuert werden kann, nach Anspruch 8, welche weiterhin eine auf der Basislage oder zwischen der Medizinbestandteilschicht und der Basislage bereitgestellte Aktivierungseinheit für ein Eindringen eines medizinischen Bestandteils umfasst, wobei die Aktivierungseinheit für ein Eindringen eines medizinischen Bestandteils einen Magneten oder einen Niederfrequenz-Generator umfasst.

10. Kompresse nach Anspruch 8 oder 9, wobei die Basislage mit einer Magnetpulverschicht als eine/die Aktivierungseinheit für ein Eindringen eines medizinischen Bestandteils ausgestattet ist.

11. Kompresse nach Anspruch 10, wobei die Magnetpulverschicht einen Gummimagneten umfasst.

12. Kompresse nach einem der Ansprüche 8 bis 11, wobei eine/die Aktivierungseinheit für ein Eindringen eines medizinischen Bestandteils zwischen der Medizinbestandteilschicht und der Basislage bereitgestellt ist.

13. Kompresse nach einem der Ansprüche 8 bis 12, wobei die Menge an Bindemittel 40% des Gesamtgewichts der gemahlenen medizinischen Bestandteile nicht übersteigt.

14. Kompresse nach einem der Ansprüche 8 bis 13, wobei das Bindemittel Honig umfasst.

## Revendications

1. Procédé de fabrication d'une compresse pour médicament à base de plantes permettant une fourniture persistante d'ingrédients médicaux à base de plantes à travers la peau ou les organes respiratoires, dans laquelle les ingrédients sont présents en différentes quantités et libérés de manière persistante jusqu'à ce que la libération de tous les ingrédients médicaux soit terminée, dans laquelle compresse la libération des ingrédients médicinaux peut être contrôlée, qui comprend : séparer deux ingrédients médicinaux ou plus prescrits ou préparés selon des effets pharmacologiques en fonction de la durée de libération des ingrédients médicinaux devant être distribués par le biais de la peau ou des organes respiratoires ; broyer les ingrédients médicinaux séparés en fines particules avec différentes tailles de 10 $\mu$m jusqu'à 400 $\mu$m en fonction de la durée de libération des ingrédients médicinaux et de leurs poids relatifs de sorte qu'un ingrédient médical de quantité inférieure a une taille supérieure et un ingrédient médical avec une quantité supérieure a une taille inférieure ; préparer un médicament à base de plantes en mélangeant les ingrédients médicinaux broyés avec un agent de liaison comprenant de la maltodextrine ou un polymère naturel ; et faire adhérer le médicament à base de plantes dans une couche d'ingrédients médicinaux à une feuille de base.

2. Procédé de fabrication d'une compresse pour médicament à base de plantes dans laquelle la libération d'ingrédients médicinaux peut être contrôlée selon la revendication 1, dans lequel ladite préparation du médicament à base de plantes comprend en outre fournir un module d'activation de perméation d'ingrédient médicinal comprenant un aimant ou un générateur de basse fréquence pour appliquer un champ magnétique ou une basse fréquence aux ingrédients médicinaux de la compresse.

3. Procédé selon la revendication 1 ou 2, dans lequel ladite feuille de base est pourvue d'une couche de poudre aimantée comme un/ledit module d'activation de perméation d'ingrédient médicinal.

4. Procédé selon la revendication 3, dans lequel ladite couche de poudre aimantée comprend un aimant en caoutchouc.

**5.** Procédé selon l'une quelconque des revendications précédentes, dans lequel un/ledit module d'activation de perméation d'ingrédient médicinal est prévu entre la couche d'ingrédients médicinaux et la feuille de base.

**6.** Procédé selon l'une quelconque des revendications précédentes, dans lequel l'agent de liaison est utilisé en une quantité ne dépassant pas 40 % (40/100) du poids total des ingrédients médicinaux broyés.

**7.** Procédé selon l'une quelconque des revendications précédentes, dans lequel ledit agent de liaison comprend du miel.

**8.** Compresse pour médicament à base de plantes permettant une fourniture persistante d'ingrédients médicaux à base de plantes à travers la peau ou les organes respiratoires, dans laquelle les ingrédients sont présents en différentes quantités et libérés de manière persistante jusqu'à ce que la libération de tous les ingrédients médicaux soit terminée, dans laquelle compresse la libération des ingrédients médicinaux peut être contrôlée, qui comprend : une feuille de base ; et une couche d'ingrédients médicinaux qui adhère à la feuille de base, dans laquelle deux ingrédients médicinaux ou plus prescrits ou préparés selon des effets pharmacologiques en fonction de la durée de libération des ingrédients médicinaux devant être distribués par le biais de la peau ou des organes respiratoires sont mélangés avec un agent de liaison comprenant de la maltodextrine ou un polymère naturel, lesquels ingrédients médicinaux sont broyés en fines particules de différentes tailles de 10 $\mu$m jusqu'à 400 $\mu$m en fonction du réglage de la durée de libération des ingrédients médicinaux et de leurs poids relatifs de sorte qu'un ingrédient médical de quantité inférieure a une taille supérieure et un ingrédient médical avec une quantité supérieure a une taille inférieure.

**9.** Compresse pour médicament à base de plantes dans laquelle la libération d'ingrédients médicinaux peut être contrôlée selon la revendication 8, qui comprend en outre un module d'activation de perméation d'ingrédient médicinal prévu sur la feuille de base ou entre la couche d'ingrédients médicinaux et la feuille de base, le module d'activation de perméation d'ingrédient médicinal comprenant un aimant ou un générateur de basse fréquence.

**10.** Compresse selon la revendication 8 ou 9, dans laquelle ladite feuille de base est pourvue d'une couche de poudre aimantée comme un/ledit module d'activation de perméation d'ingrédient médicinal.

**11.** Compresse selon la revendication 10, dans laquelle ladite couche de poudre aimantée comprend un aimant en caoutchouc.

**12.** Compresse selon l'une quelconque des revendications 8 à 11, dans laquelle un/ledit module d'activation de perméation d'ingrédient médicinal est prévu entre la couche d'ingrédients médicinaux et la feuille de base.

**13.** Compresse selon l'une quelconque des revendications 8 à 12, dans laquelle la quantité d'agent de liaison n'est pas supérieure à 40 % du poids total des ingrédients médicinaux broyés.

**14.** Compresse selon l'une quelconque des revendications 8 à 13, dans laquelle ledit agent de liaison comprend du miel.

【Fig. 1】

【Fig. 2】

```
┌─────────────────────────────────────┐
│   Separating medicinal ingredients   │  ⟋ S1
└─────────────────────────────────────┘
                  │
                  ▼
┌─────────────────────────────────────┐
│     Grinding medicinal ingredients    │  ⟋ S2
└─────────────────────────────────────┘
                  │
                  ▼
┌─────────────────────────────────────┐
│       Preparing herbal medicine       │  ⟋ S3
└─────────────────────────────────────┘
                  │
                  ▼
┌─────────────────────────────────────┐
│       Adhering herbal medicine        │  ⟋ S4
└─────────────────────────────────────┘
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2493155 A
- US 4141359 A
- US 4250878 A
- US 3163166 A
- US 4166457 A
- US 4273135 A
- US 3289671 A
- US 4239052 A
- US 4367745 A
- US 3677268 A
- US 4243052 A
- US 588479 A
- KR 0775675
- CN 101278979 A